Europäisches Patentamt

European Patent Office

Office européen des brevets

(11) Publication number: **0 137 063**
**B1**

(12) **EUROPEAN PATENT SPECIFICATION**

(45) Date of publication of patent specification: **22.07.87**

(51) Int. Cl.⁴: **C 07 C 29/76, C 07 C 31/26**

(21) Application number: **83110067.2**

(22) Date of filing: **08.10.83**

(54) **Bulk separation of polyhydric alcohols by selective adsorption on zeolitic molecular sieves.**

(43) Date of publication of application:
**17.04.85 Bulletin 85/16**

(45) Publication of the grant of the patent:
**22.07.87 Bulletin 87/30**

(84) Designated Contracting States:
**AT BE CH DE FR GB IT LI LU NL SE**

(56) References cited:
**BE-A- 837 201**
**GB-A- 877 643**
**US-A-4 283 560**

**RECUEIL, JOURNAL OF THE ROYAL NETHERLANDS CHEMICAL SOCIETY, vol. 97, no. 6, June 1978, pages 156-158 TH.M. WORTEL et al.: "Carbohydrate separation by X-zeolites. Cation and solvent effects"**

(73) Proprietor: **UNION CARBIDE CORPORATION**
**39 Old Ridgebury Road**
**Danbury Connecticut 06817 (US)**

(72) Inventor: **Sherman, John Delano**
**91 Valley View Road**
**Chappaqua, NY 10514 (US)**
Inventor: **Chao, Chien Chung**
**Glenwood Road**
**Millwood, NY 10546 (US)**

(74) Representative: **Eggert, Hans-Gunther, Dr.**
**Räderscheidtstrasse 1**
**D-5000 Köln 41 (DE)**

## Description

The present invention relates to the liquid phase separation of mannitol from sorbitol and/or mannitol from galactitol. More particularly, it relates to such a separation by selective adsorption on certain types of zeolite molecular sieves.

Mannitol, sorbitol and galactitol are polyhydric alcohols made by the reduction of sugars. Very often, the reduction reaction products are mixtures of these polyhydric alcohols. Mannitol and sorbitol are especially important commercial polyhydric alcohols in their pure forms.

Mannitol may be produced from invert sugar, fructose or from mannose. In the first instance, sucrose is hydrolyzed to "invert sugar", which is a mixture of glucose and fructose. This mixture is reduced, resulting in a mixture of sorbitol and mannitol in a ratio of approximately 3:1. It is necessary to separate the mannitol from the sorbitol in the mixture in order to obtain a pure product. In general, according to "Specialized Sugars for the Food Industry," by J. P. Johnson, Noyes Data Corporation, Parkridge, New Jersey (1976), this is done by successive recrystallizations, an expensive and time-consuming process.

Another method to separate mannitol from sorbitol in admixture is disclosed in United States Patent No. 3,864,406 (Melaja et al.). Melaja et al. teaches the use of the calcium exchanged form of a sulfonated polystyrene cation exchange resin which is crosslinked with divinylbenzene.

Mannitol may also be produced by the hydrogenation of mannose or sources of mannose such as pulping liquors and other hydrolyzates of hemicellulose. Hydrogenation of mannose results in the production of pure mannitol. However, these lignocellulosic sources also contain wood sugars, some of which are hard to separate from mannose. As a result, hydrogenation products of these wood sugars, in particular galactose, must be separated from mannitol in order to obtain a pure product.

Crystallization-type separations of mannitol from galactitol have been disclosed in United States Patent No. 3,944,625 (J. A. Neal). According to that process, the mannitol-galactitol admixture is dissolved in a mixed solvent of alkanol and water, and small amounts of soluble salts of iron, nickel or cobalt are added.

The use of resins in separating sugar alcohols has been found to have several disadvantages compared to the use of zeolite molecular sieves. First, the resins must be used as small beads, and the fine of the resin may be carried through valves and jam them. Also, resins tend to swell when exposed to aqueous solutions. This diminishes the precision of the separations because the volume of resin changes from the beginning to the end of each process run. The zeolite molecular sieves used in this invention do not swell when they encounter water and, therefore, flow rates and volumes can remain uniform throughout the process. another potential disadvantage to using polystyrene resins is due to their organic nature. It is therefore an object of this invention to provide an efficient process for the separation of sugar alcohols.

It is also an object of this invention to provide a precise process for separating mannitol from galactitol and/or from sorbitol.

It is a further object of this invention to provide a process for separation of sugar alcohols by selective absorption on inorganic zeolite molecular sieves.

It has been discovered that certain zeolites are well-suited to separating mannitol from sorbitol and mannitol from galactitol.

### Brief description of figures

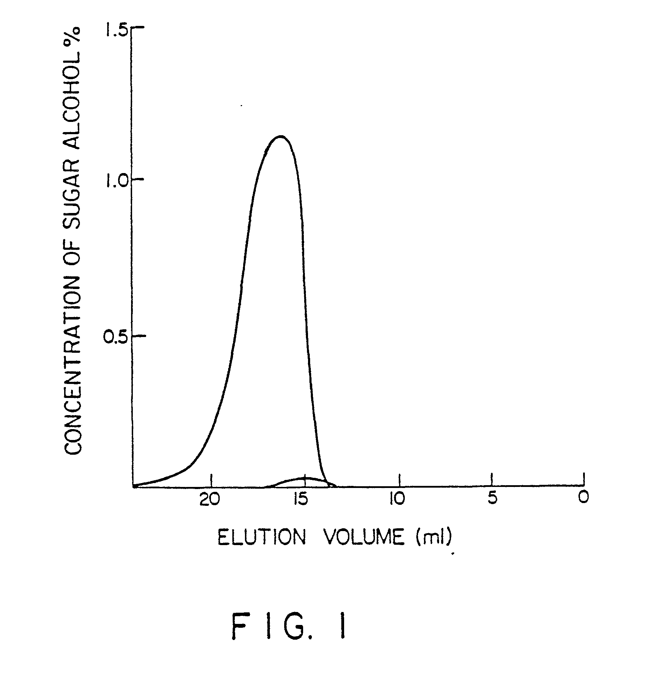

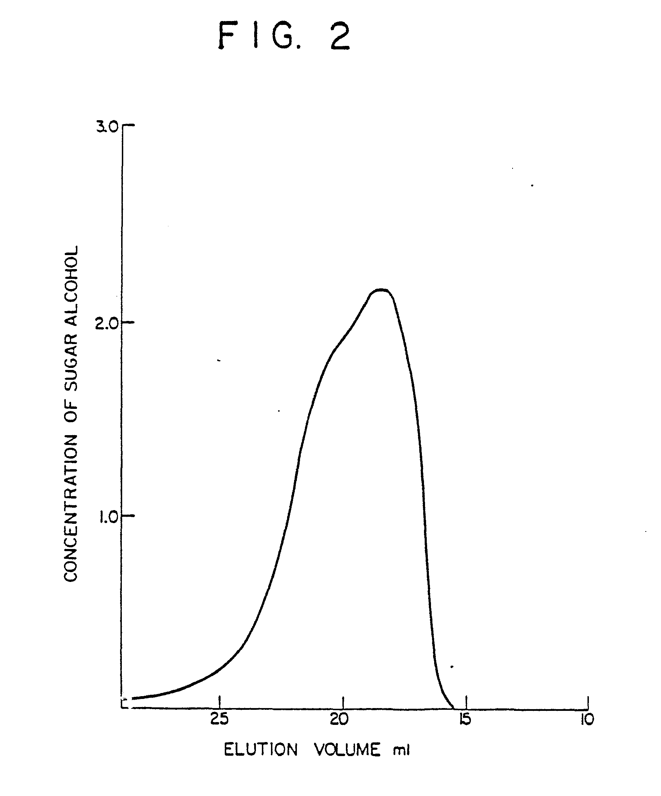

Figures 1 and 2 show elution curves of polyhydric alcohols where the separation medium is potassium-substituted zeolite Type Y.

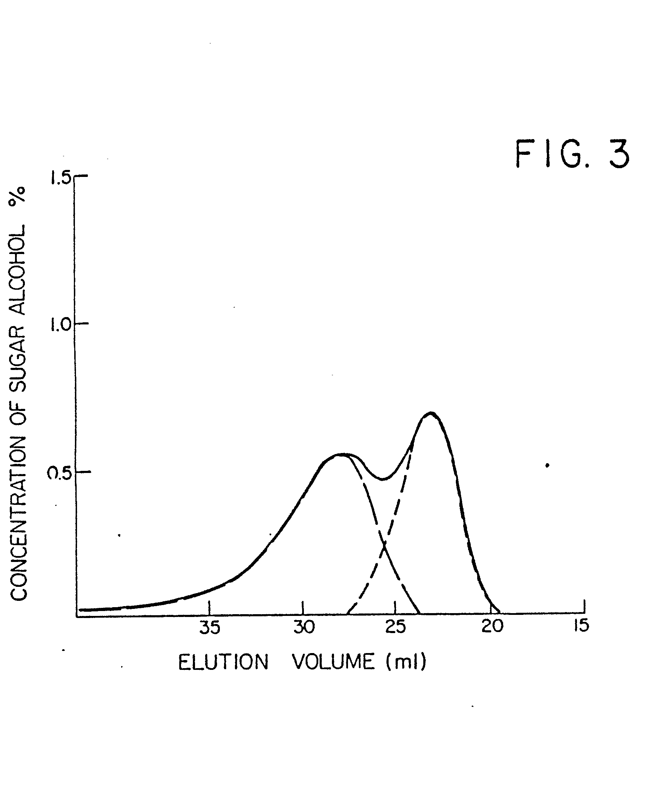

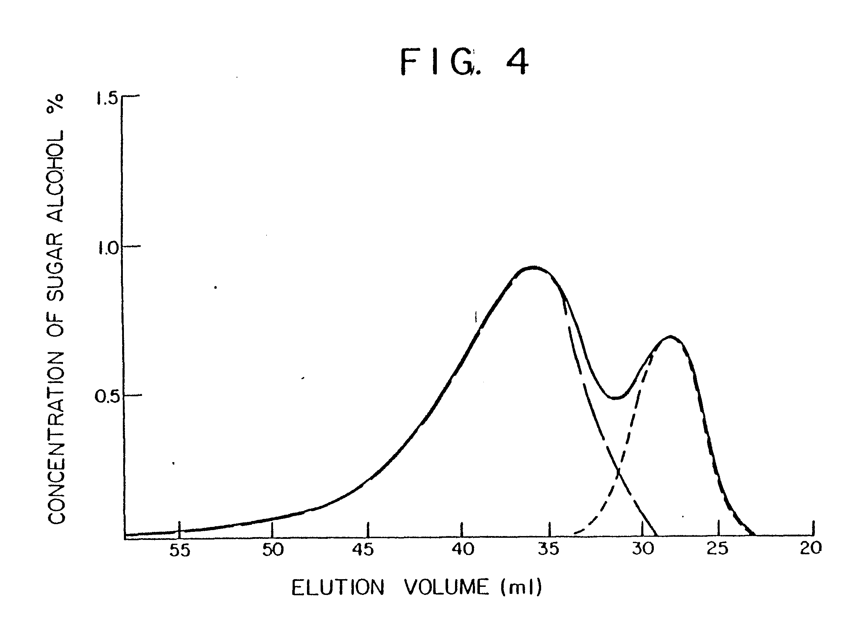

. Figures 3 and 4 show elution curves of polyhydric alcohols where the separation medium is barium-substituted zeolite Type X.

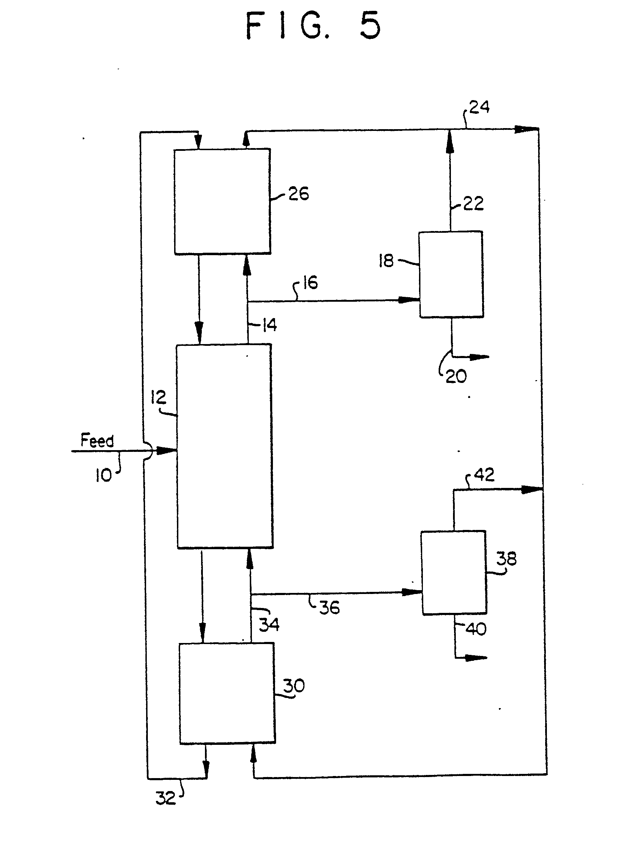

Figure 5 shows one method in which the process of this application may be employed.

### Description of the invention

The present invention comprises a process for the separation of mixtures of polyhydric alcohols, said mixtures comprising mannitol and one or more members of the group consisting of sorbitol and galactitol, by selective adsorption which comprises contacting a mixture comprising said compounds at a temperature of from 30°C to 100°C and at a pressure sufficient to maintain the system in the liquid phase with an adsorbent composition comprising at least one crystalline aluminosilicate zeolite selected from a group consisting of zeolite type Y and zeolite type X wherein the zeolitic cations which occupy more than 53% the available cation sites are selected from the group consisting of barium and calcium, with exception of calcium zeolite X; whereby a polyhydric alcohol is selectively adsorbed thereon, removing the non-adsorbed portion of said mixture from contact with the zeolite adsorbent; and desorbing the adsorbate therefrom by contacting said adsorbent with a desorbing agent and recovering the desorbed polyhydric alcohol.

Zeolite Y and the method for its manufacture are described in detail in United States Patent No. 3,130,007, issued April 21, 1964 to D. W. Breck. Zeolite X and the method for its manufacture are described in detail in United States Patent No. 2,882,244, issued April 14, 1959 to R. M. Milton.

The adsorption affinities of various zeolites for sugars was determined by a "pulse test". This test consisted of packing a column with the appropriate zeolite, placing it in a block heater to maintain constant temperature, and eluting solutions through the column with water to determine retention volume of solute. The retention volume of solute is defined as elution volume of solute minus "void volume". "Void volume" is the volume of solvent needed to elute a non-sorbing solute through the column. A soluble polymer of fructose, inulin, which is too large to be sorbed into the zeolite pores, was chosen as the solute to determine void volume. The elution volume of inulin was first determined. The elution volumes of sugars were then determined under similar experimental conditions. The retention volumes were calculated and are recorded in Table I, below. From the retention volume data, the separation factors (S.F.),

$$\alpha \frac{\text{Mannitol}}{\text{Galactitol}} \text{ (S.F.}_{M/G}) \text{ and } \alpha \frac{\text{Sorbitol}}{\text{Mannitol}} \text{ (S.F.}_{S/M}), \text{ where}$$

$$\text{M/G stands for } \frac{\text{Mannitol}}{\text{Galactitol}} \text{ and S/M stands for } \frac{\text{Sorbitol}}{\text{Mannitol}}$$

were calculated in accordance with the following equations:

$$S.F._{M/G} = \alpha(\text{mannitol/galactitol})$$

$$= \frac{(\text{retention volume for mannitol peak})}{(\text{retention volume for galactitol peak})}$$

$$S.F._{S/M} = \alpha(\text{sorbitol/mannitol})$$

$$= \frac{(\text{retention volume for sorbitol peak})}{(\text{retention volume for mannitol peak})}$$

A $S.F._{M/G}$ factor greater than unity indicates that the particular adsorbent was selective for mannitol over galactitol. A $S.F._{S/M}$ greater than unity indicates that the particular adsorbent was selective for sorbitol over mannitol. The separation factor values calculated according to the above-mentioned method are found in Table II.

In order to show more dramatically the ability to separate sugar alcohols, Table II includes a column showing the difference between the -value and 1.00 multiplied by one hundred. This clearly indicates each zeolite's ability to perform the desired separations. As known from Recueil, Journal of the Royal Netherlands Chemical Society, vol. 97, No. 6, June 1978, pages 156—158, attempted separation of sorbitol and mannitol on NaCaX-65% was unsuccessful. Table II discloses separation factors nearly the same for the separation of mannitol from galactitol or sorbitol.

## TABLE I
### Retention volume of sugar alcohols (in ml)

Column dimension:     40 cm×0.77 cm I.D.
Zeolite form:     Powder except where indicated
Flow rate:     1 ml/min
Temperature:     160°F

| Zeolite | Inulin | Galactitol | Mannitol | Sorbitol |
|---------|--------|-----------|----------|----------|
| KX | 0 | | 3.0 | 2.8 |
| KY | 0 | 1.4 | 1.3 | 1.2 |
| NaX | 0 | | 2.3 | 2.3 |
| NaY | 0 | 1.5 | 1.7 | 1.8 |
| *LiY | 0 | | 2.5 | 2.5 |
| BaX | 0 | 8.7 | 13.6 | 21.7 |
| *BaY | 0 | 13.0 | 15.0 | 12.3 |
| *SrX | 0 | −7.2 | 7.2 | 8.0 |
| *SrY | 0 | 11.5 | 12.0 | 11.3 |
| CaX | 0 | −7 | 6.7 | 6.8 |
| CaY | 0 | 10.1 | 11.6 | 13.1 |

* Zeolite in mesh form.

## TABLE II
### Separation factors of sugar alcohol separations

| Zeolite | Mannitol / Galactitol (α) | α−1×100 | Sorbitol / Mannitol (α) | α−1×100 |
|---------|------|---------|------|---------|
| KX | | | 0.93 | 7 |
| KY | −1.0 | 0 | 0.92 | 8 |
| NaX | | | 1.00 | 0 |
| NaY | 1.13 | 13 | 1.06 | 6 |
| LiY | | | 1.0 | 0 |
| BaX | 1.5 | 60 | 1.6 | 60 |
| BaY | 1.15 | 15 | 0.82 | −18 |
| SrX | 0 | 0 | 1.11 | 11 |
| SrY | 1.04 | 4 | 0.94 | −6 |
| CaX | 1.0 | 0 | 1.01 | 1 |
| CaY | 1.15 | 15 | 1.13 | 13 |

In separating mannitol from galactitol or sorbitol in the present process, a bed of solid zeolite

4

adsorbent is preferentially loaded with adsorbates, the unadsorbed or raffinate mixture is removed from the adsorbent bed, and the adsorbed sugar alcohol is then desorbed from the zeolite adsorbent by a desorbant. The adsorbent can, if desired, be contained in a single bed, a plurality of beds in which conventional swing-bed operation techniques are utilized, or a simulated moving-bed countercurrent type of apparatus. A preferred mode of operation is the simulated moving-bed technique, for example, that described in United States Patent No. 2,985,589 issued May 23, 1961 to D. B. Broughton et al. In this method of operation, the selection of a suitable displacing or desorbing agent or fluid (solvent) must take into account the requirements that it be capable of readily displacing adsorbed alcohol from the adsorbent bed and also that alcohol from the feed mixture be able to displace adsorbed desorbing agent from a previous desorption step. Further, the desorbing agent employed should be readily separable from admixture with the sugar alcohol components of the feedstock. Therefore it is contemplated that a desorbing agent having characteristics which allow it to be easily fractionated from the sugar alcohol should be used. For example, volatile desorbing agents should be used, such as alcohols, ketones, admixtures of alcohols and water, particularly methanol and ethanol. The most preferred desorbing agent is water.

While it is possible to utilize the activated adsorbent zeolite crystals in a non-agglomerated form, it is generally more feasible, particularly when the process involves the use of a fixed adsorption bed, to agglomerate the crystals into larger particles to decrease the pressure drop in the system. The particular agglomerating agent and the agglomeration procedure employed are not critical factors, but it is important that the bonding agent be as inert toward the sugar alcohols and desorbing agent as possible. The proportions of zeolite and binder are advantageously in the range of 4 to 20 parts zeolite per part binder on an anhydrous weight basis.

The temperature at which the adsorption step of the process should be carried out should be from about 30°C to the boiling point of the sugar alcohol solution (about 110°C). It has been found that at temperatures below about 30°C the counter-diffusion rate between sugar alcohols is too slow, i.e., a sufficient selectivity for the sorbitol or mannitol is not exhibited by the zeolite. As the temperature increases, the temperature at which the desorbent boils will be reached. Preferably, the adsorption step should take place between about 50°C and about 90°C, most preferably between 60° and 80°C. Pressure conditions must be maintained so as to keep the system in liquid phase. High process temperatures needlessly necessitate high pressure apparatus and increase the cost of the process.

The preferable method for practicing the process of this invention is separation by chromatographic column. In this method, a sugar alcohol solution containing the mixture of sugar alcohols to be separated is injected for a short period of time at the top of the column and eluted down through the column with water. As the mixture passes through the column, chromatographic separation leads to a zone increasingly enriched in the adsorbed sugar alcohol. The degree of separation increases as the mixture passes further down through the column until a desired degree of separation is achieved. At this point, the effluent from the column is first shunted to one receiver which collects a pure product. Next, during the period of time when there is a mixture of sugar alcohols emerging from the column, the effluent is directed towards a "receiver for mixed product". Next, when the zone of adsorbed alcohol emerges from the end of the column, the effluent is directed to a receiver for that product.

As soon as the chromatographic bands have passed far enough through the column, a new slug is introduced at the entrance of the column and the whole process cycle is repeated. The mixture which exits from the end of the column between the times of appearance of the pure fractions is recycled back to the feed and passed through the column again, to extinction.

The degree of separation of the two peaks as they pass through this chromatographic column will increase as the column length is increased. Therefore, one can design a column of sufficient length to provide any desired degree of separation of the two components from each other, even where the degree of overlap of the two peaks is the greatest.

Therefore, it is also possible to operate such a process in a mode which will involve essentially no recycle of an unseparated mixture back to the feed. However, if high purities are required such a high degree of separation may require an exceptionally long column. In addition, as the components are eluted through the column their average concentrations gradually decline. In the case of the sugar alcohols being eluted with water, this would mean that the product streams would be increasingly diluted with water. Therefore, it is highly likely that an optimum process (to achieve high degrees of purity of the two components) should involve the use of a much shorter column (than would be required for complete separation of the peaks) and also involve separating out the portion of the effluent containing the mixture of both peaks and recycling it to feed, as discussed above.

Another method for practicing the process of this invention is illustrated by the drawing in Figure 5. In this method, a number of fixed beds are connected to one another by conduits which are also connected to a special valve. The valve subsequentially moves the liquid feed and product takeoff points to different positions around a circular array of the individual fixed beds in such a manner as to simulate countercurrent motion of the adsorbent. This process is well-suited to binary separations.

In the drawings, Figure 5 represents a hypothetical moving-bed countercurrent flow diagram involved in carrying out a typical process embodiment of the present invention.

With reference to the drawing, it will be understood that whereas the liquid stream inlets and outlets are represented as being fixed, and the adsorbent mass is represented as moving with respect to the counter flow of feedstock and desorbing material, this representation is intended primarily to facilitate describing the functioning of the system. In practice the sorbent mass would ordinarily be in a fixed bed with the liquid stream inlets and outlets moving with respect thereto. Accordingly, a feedstock comprising a mixture of mannitol with sorbitol and/or galactitol is fed into the system through line 10 to adsorbent bed 12 which contains particles of regenerated zeolite BaX or BaY adsorbent in transit downwardly therethrough. The temperature is at 70°C throughout the entire system and the pressure is substantially atmospheric. The component of the feedstock is adsorbed preferentially on the zeolite particles moving through bed 12, and the raffinate is entrained in the liquid stream of water desorbing agent leave bed 12 through line 14 and a major portion thereof is withdrawn through line 16 and fed into evaporation apparatus 18 wherein the mixture is fractionated and the concentrated raffinate is discharged through line 20. The water desorbing agent leaves the evaporation apparatus 18 through line 22 and is fed to line 24 through which it is admixed with additional desorbing agent leaving the adsorbent bed 26, and is recycled to the bottom of adsorbent bed 30. The zeolite BaX or BaY carrying adsorbed sugar alcohol passes downward through line into bed 30 where it is counter-currently contacted with recycled desorbing agent which effectively desorbs the sugar alcohol therefrom before the adsorbent passes through bed 30 and enters line 32 through which it is recycled to the top of adsorbent bed 26. The desorbing agent and desorbed sugar alcohol level 30 through line 34. A portion of this liquid mixture is diverted through line 36, where it passes evaporation apparatus 38, and the remaining portion passes upward through adsorbent bed 12 for further treatment as hereinbefore described. In evaporation apparatus 38, the desorbing agent and sugar alcohol are fractionated. The sugar alcohol product is recovered through line 40 and the desorbing agent is either disposed of or passed through line 42 into line 24 for recycle as described above. The undiverted portion of the desorbing agent/raffinate mixture passes from bed 12 through line 14 enters bed 26 and moves counter-currently upward therethrough with respect to the desorbing agent-laden zeolite adsorbent passing downwardly therethrough from recycle line 32. The desorbing agent passes from bed 26 in a relatively pure form through recycle line 24 and to bed 30 as hereinbefore described.

The following examples are illustrative of the practice of this invention. However, they do not limit the invention to the embodiments in the Examples.

As used in the Examples appearing below the following abbreviations and symbols have the indicated meaning:

KY     Potassium-exchanged zeolite Y
BaX     Barium-exchanged zeolite X
gpm/ft$^2$ gallons per minute per square foot.

Example 1

A 40-centimeter column having an inside diameter of 0.77 centimeters was loaded with KY powder zeolite. The column was filled with water and maintained at a temperature of 160°F. Water was then pumped through the column and a flow rate of 0.53 gpm/ft$^2$ was maintained. For a period of one minute a solution containing 2 weight percent galactitol and 2 weight percent mannitol ("feed pulse") was substituted for the water stream. After the one-minute feed pulse, the water feed which contained no dissolved sugar alcohols was reestablished. The composition of the effluent stream was monitored by a refractive index detector. The elution curve is given in Figure 1. Figure 1 shows that galactitol and mannitol are not separated using KY.

Example 2

The same column and experimental procedures used in Example 1 were used. However, this composition of the feed pulse solution was changed to 3% mannitol and 7% sorbitol. The elution curve is given in Figure 2. This shows that mannitol and sorbitol are not separated using KY.

Example 3

The same experimental procedures used in Example 1 were employed. However, the zeolite was changed to BaX. The feed pulse was 2% galactitol and 2% mannitol. The elution curve is shown in Figure 3. Figure 3 shows that galactitol and mannitol are separated using BaX, galactitol being eluted first.

Example 4

The same column and experimental procedures used in Example 3 were employed. However, the feed pulse solution was changed to one containing 3% mannitol and 7% sorbitol. Figure 4, showing the elution curve, indicates that BaX separates mannitol from sorbitol.

**Claims**

1. A process for the separation of mixtures of polyhydric alcohols, said mixtures comprising mannitol and one or more members of the group consisting of sorbitol and galactitol, by selective adsorption which comprises contacting a mixture comprising said compounds at a temperature of from 30°C to 110°C and at a pressure sufficient to maintain the system in the liquid phase with an adsorbent composition comprising at least one crystalline aluminosilicate zeolite selected from a group consisting of zeolite type X and zeolite type Y in which the zeolitic cations which occupy more than 55% of the available cation sites are selected from the group consisting of barium and calcium, with the exception of calcium zeolite X; whereby a polyhydric alcohol is selectively adsorbed thereon, removing the non-adsorbed portion of said mixture from contact with the zeolite adsorbent and desorbing the adsorbate therefrom by contacting said adsorbent with a desorbing agent and recovering the desorbed polyhydric alcohol.

2. A process in accordance with claim 1 wherein the temperature is from about 30°C to about 90°C.

3. A process in accordance with claim 1 wherein the temperature is from about 60°C to about 80°C.

4. A process in accordance with claim 1 wherein the desorbent is selected from the group consisting of alcohols, ketones and water.

5. A process in accordance with claim 1 wherein the desorbent is water.

6. A process for separating mannitol from galactitol by selective adsorption which comprises contacting a mixture comprising said compounds at a temperature of from about 30°C to 110°C and at a pressure sufficient to maintain the system in the fluid phase with an adsorbent composition comprising at least one crystalline aluminosilicate zeolite of type X in which the zeolitic cations which occupy more than 55% of the available cation sites are barium; whereby the mannitol is selectively adsorbed thereon, removing the non-adsorbed portion of said mixture from contact with the zeolite adsorbent, and desorbing the mannitol therefrom by contacting said adsorbent with a desorbing agent and recovering the desorbed mannitol.

7. A process for separating mannitol from galactitol by selective adsorption which comprises contacting a mixture comprising said compounds at a temperature of from about 30°C to 110°C and at a pressure sufficient to maintain the system in the fluid phase with an adsorbent composition comprising at least one crystalline aluminosilicate zeolite of type Y in which the zeolitic cations which occupy more than 55% of the available cation sites are barium; whereby the mannitol is selectively adsorbed thereon, removing the non-adsorbed portion of said mixture from contact with the zeolite adsorbent, and desorbing the mannitol therefrom by contacting said adsorbent with a desorbing agent and recovering the desorbed mannitol.

8. A process for separating sorbitol from mannitol by selective adsorption which comprises contacting a mixture comprising said compounds at a temperature of from about 30°C to 110°C and at a pressure sufficient to maintain the system in the fluid phase with an adsorbent composition comprising at least one crystalline aluminosilicate zeolite of type X in which the zeolitic cations which occupy more than 55% of the available cation sites are barium; whereby the sorbitol is selectively adsorbed thereon, removing the non-adsorbed portion of said mixture from contact with the zeolite adsorbent, and desorbing the sorbitol therefrom by contacting said adsorbent with a desorbing agent and recovering the desorbed sorbitol.

**Patentansprüche**

1. Verfahren zur Auftrennung von Mischungen von Polyhydroxyalkoholen, wobei diese Mischungen Mannit und ein oder zwei Vertreter der Gruppe bestehend aus Sorbit und Galactit enthalten, mittels selektiver Adsorption, die umfaßt: In Kontakt bringen einer Mischung, die diese Verbindungen enthält, bei einer Temperatur von zwischen 30°C und 110°C und einem Druck, der ausreichend ist, um das System in der Flüssigphase zu halten mit einer Adsorbens-Zusammensetzung, die wenigstens einen kristallinen Aluminosilikat-Zeolith enthält, ausgewählt aus der Gruppe bestehend aus den Typ X-Zeolithen und den Typ Y-Zeolithen, bei denen die zeolithischen Kationen, die mehr als 55% der verfügbaren Kationenplätze besetzen, ausgewählt werden aus der Gruppe bestehend aus Barium und Calcium, mit Ausnahme des Calcium-Zeoliths X, wobei ein Polyhydroxyalkohol selektiv auf dieser Adsorbens-Zusammensetzung adsorbiert wird, Entfernen des nicht adsorbierten Anteils aus dem Zeolith-Adsorbens und Desorbieren des Adsorbats aus diesem durch Kontaktbringen dieses Adsorbens mit einem Desorbierungsagens und Gewinnung des desorbierten Polyhydroxyalkohols.

2. Verfahren gemäß Anspruch 1, bei dem die Temperatur zwischen ungefähr 50°C und ungefähr 90°C liegt.

3. Verfahren gemäß Anspruch 1, bei dem die Temperatur zwischen ungefähr 60°C und ungefähr 80°C liegt.

4. Verfahren gemäß Anspruch 1, bei dem das Desorbens ausgewählt wird aus der Gruppe bestehend aus Alkoholen, Ketonen und Wasser.

5. Verfahren gemäß Anspruch 1, bei dem das Desorbens Wasser ist.

6. Verfahren zur Trennung von Mannit und Galactit durch selektive Adsorption, die darin besteht, eine Mischung, die diese Verbindungen enthält bei einer Temperatur zwischen ungefähr 30°C und 110°C und einem Druck, der ausreicht, das System in der Flüssigphase zu halten, mit einer Adsorbens-Zusammensetzung in Kontakt zu bringen, die wenigstens einen kristallinen Aluminosilikat-Zeolith des Typs X enthält, in dem die zeolithischen Kationen, die mehr als 55% der verfügbaren Kationenplätze besetzen, Bariumionen sind, wobei Mannit selektiv auf der Adsorbens-Zusammensetzung adsorbiert wird, Entfernen des nicht adsorbierten Anteils dieser Mischung aus dem Zeolith-Adsorbens und Desorbieren des Mannits aus diesem durch in Kontakt bringen des Adsorbens mit einem desorbierenden Agens und Gewinnung des desorbierten Mannits.

# 0 137 063

7. Verfahren zur Trennung von Mannit und Galactit durch selektive Adsorption, die darin besteht, eine Mischung, die diese Verbindungen enthält bei einer Temperatur von zwischen ungefähr 30°C und 110°C und einem Druck, der ausreichend ist um das System in der Flüssigphase zu halten mit einer Adsorbens-Zusammensetzung in Kontakt zu bringen, die wenigstens einen kristallinen Alumosilikat-Zeolith des Y-Typs enthält, in dem die zeolithischen Kationen, die mehr als 55% der verfügbaren Kationenplätze besetzen, Barium-Ionen sind, wobei Mannit selektiv auf dem Adsorbens adsorbiert wird, Entfernen des nichtadsorbierten Anteils dieser Mischung aus dem Zeolith-Adsorbens und Desorbieren des Mannits aus diesem durch in Kontakt bringen dieses Adsorbens mit einem desorbierenden Agens und Gewinnung des desorbierten Mannits.

8. Verfahren zur Trennung von Sorbit und Mannit durch selektive Adsorption, umfassend das in Kontakt bringen einer Mischung, die diese Verbindungen enthält bei einer Temperatur von zwischen ungefähr 30°C und 110°C und einem Druck, der ausreichend ist um das System in der Flüssigphase zu halten, mit einer Adsorbens-Zusammensetzung, die wenigstens einen kristallinen Aluminosilikat-Zeolith des X-Typs enthält, bei dem die zeolithischen Kationen, die mehr als 55% der verfügbaren Kationenplätze besetzen, Barium-Ionen sind, wobei das Sorbit selektiv auf diesem adsorbiert wird, Entfernen des nicht-adsorbierten Anteils dieser Mischung aus dem Zeolith-Adsorbens und Desorbieren des Sorbits aus diesem durch in Kontakt bringen dieses Adsorbens mit einem desorbierenden Agens und Gewinnung des desorbierten Sorbits.

## Revendications

1. Procédé de séparation de mélanges d'alcools polyhydroxyliques, lesdits mélanges comprenant le mannitol et un ou plusieurs représentants du groupe comprenant le sorbitol et le galactitol, par adsorption sélective, qui consiste à mettre en contact un mélange comprenant lesdits composés à une température de 30°C à 110°C et sous une pression suffisante pour maintenir le système en phase liquide avec une composition adsorbante comprenant au moins une zéolite du type aluminosilicate cristallin choisie entre la zéolite de type X et la zéolite de type Y, dans laquelle les cations zéolitiques qui occupent plus de 55% des sites cationiques disponibles sont choisis entre le baryum et le calcium, à l'exception de la zéolite X calcique; permettant ainsi l'adsorption sélective d'un alcool polyhydroxylique à la surface de celle-ci, à supprimer le contact de la portion non adsorbée dudit mélange avec l'adsorbant zéolitique et à désorber l'adsorbat de celui-ci par mise en contact dudit adsorbant avec un agent désorbant et à recueillir l'alcool polyhydroxylique désorbé.

2. Procédé suivant la revendication 1, dans lequel la température est comprise entre environ 50°C et environ 90°C.

3. Procédé suivant la revendication 1, dans lequel la température est comprise entre environ 60°C et environ 80°C.

4. Procédé suivant la revendication 1, dans lequel le désorbant est choisi dans le groupe comprenant des alcools, des cétones et l'eau.

5. Procédé suivant la revendication 1, dans lequel le désorbant est l'eau.

6. Procédé de séparation du mannitol du galactitol par adsorption sélective, qui consiste à mettre en contact un mélange comprenant lesdits composés à une température d'environ 30°C à 110°C et sous une pression suffisante pour maintenir le système en phase fluide avec une composition adsorbante comprenant au moins une zéolite du type aluminosilicate cristallin de type X dans laquelle les cations zéolitiques qui occupent plus de 55% des sites cationiques disponibles sont les cations baryum, permettant ainsi l'adsorption sélective du mannitol sur celle-ci, à supprimer le contact de la portion non adsorbée dudit mélange avec l'adsorbant zéolitique, et à désorber le mannitol de celui-ci par mise en contact dudit adsorbant avec un agent désorbant, et à recueillir le mannitol désorbé.

7. Procédé de séparation du mannitol du galactitol par adsorption sélective, qui consiste à mettre en contact un mélange comprenant lesdits composés à une température d'environ 30°C à 110°C et sous une pression suffisante pour maintenir le système en phase fluide avec une composition adsorbante comprenant au moins une zéolite du type aluminosilicate cristallin de type Y dans laquelle les cations zéolitiques qui occupent plus de 55% des sites cationiques disponibles sont les cations barym; permettant ainsi l'adsorption sélective du mannitol sur celle-ci, à supprimer le contact de la portion non adsorbée dudit mélange avec l'adsorbant zéolitique, et à désorber le mannitol de celui-ci par mise en contact dudit adsorbant avec un agent désorbant, et à recueillir le mannitol désorbé.

8. Procédé de séparation du sorbitol du mannitol par adsorption sélective, qui consiste à mettre en contact un mélange comprenant lesdits composés à une température d'environ 30°C à 110°C et sous une pression suffisante pour maintenir le système en phase fluide avec une composition adsorbante comprenant au moins une zéolite du type aluminosilicate cristallin de type X dans laquelle les cations zéolitiques qui occupent plus de 55% des sites cationiques disponibles sont les cations baryum; permettant ainsi l'adsorption sélective du sorbitol sur celle-ci, à supprimer le contact de la portion non adsorbée dudit mélange avec l'adsorbant zéolitique, et à désorber le sorbitol de celui-ci par mise en contact dudit adsorbant avec un agent désorbant, et à recueillir le sorbitol désorbé.

FIG. I

1

FIG. 2

0 137 063

2

FIG. 3

FIG. 4

0 137 063

# F I G. 5